# EUROPEAN PATENT APPLICATION

(11) **EP 4 736 851 A1**
(43) Date of publication of application: **06.05.2026**
(21) Application number: 24830564.1
(22) Date of filing: 18.06.2024
(51) Int. Cl.: A61K 31/198, A61K 47/02, A61K 31/165, A61K 9/20, A61P 25/16

(54) **LEVODOPA-BENSERAZIDE HYDROCHLORIDE COMPOSITION AND PREPARATION THEREFOR**

(30) Priority: 27.06.2023 CN 202310766123
(71) Applicant: Zhejiang Huahai Pharmaceutical Co., Ltd., Taizhou, Zhejiang 317024 (CN)
(72) Inventor: ZHU, Lulu, Zhejiang 317024 (CN); ZHANG, Zhengzan, Zhejiang 317024 (CN); LOU, Guichuan, Zhejiang 317024 (CN); SHEN, Jiaxu, Zhejiang 317024 (CN); JIANG, Shiyong, Zhejiang 317024 (CN); LU, Jinping, Zhejiang 317024 (CN); ZHAO, Zhouming, Zhejiang 317024 (CN); GUO, Xiaodi, Zhejiang 317024 (CN)
(74) Representative: Graf von Stosch Patentanwaltsgesellschaft mbH
(86) International application number: PCT/CN2024/099808
(87) International publication number: WO 2025/001918

(57) **Abstract**

Provided in the present invention are a pharmaceutical composition used for treating Parkinson's disease and a preparation method therefor. Tablets prepared from the pharmaceutical composition have better stability, and involve a simple production process, thus facilitating commercial production.

## Description

The present application claims the priority of Chinese Patent Application No. CN202310766123.5, filed on June 27, 2023, which is incorporated herein by reference in its entirety.

### TECHNICAL FIELD

The present application belongs to the field of pharmaceutical technology, in particular to a co-beneldopa pharmaceutical composition and preparation therefor.

### BACKGROUND

Parkinson's disease (PD) is the second most common neurodegenerative disease in department of neurology, after Alzheimer's disease. It is estimated that there were up to 9.4 million patients worldwide in 2020. The main pathological changes are degeneration and death of midbrain substantia nigra dopaminergic neurons, a significant decrease of dopamine (DA) content in striatum, and an appearance of eosinophilic inclusion bodies, i.e. Lewy bodies, in the cytoplasm of residual neurons of the substantia nigra.

Among the various treatment methods for Parkinson's disease, drug therapy is still the most effective. By maintaining the balance of the two neurotransmitters, dopamine and acetylcholine, in the striatum, the clinical symptoms are improved. The drugs mainly include levodopa, DA receptor agonists, MAO-B inhibitors, COMT inhibitors, and the like.

Co-Beneldopa is a combination of levodopa and benserazide hydrochloride in a ratio of 4:1. It was first approved for marketing in Europe in 1973 and subsequently launched in many countries, including Japan and China. As a classic compound for the treatment of Parkinson's disease and Parkinson plus syndrome, it has been used clinically for many years with definite efficacy and low cost.

Wherein the chemical name of benserazide hydrochloride is 2-[(2,3,4-trihydroxyphenyl)methyl]hydrazide-DL-serine hydrochloride. Its chemical structural formula is as follows:

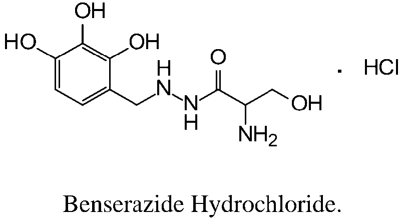

Benserazide hydrochloride is unstable under alkaline, light, and humidity-heat conditions, and is particularly unstable under humid-heat conditions. As temperature or humidity increases, the degradation of benserazide accelerates, primarily yielding the following two degradation impurities: Impurity A: (2RS)-2-amino-3-hydroxypropanehydrazide, and Impurity B: (2RS)-2-amino-3-hydroxy-N,N-bis(2,3,4-trihydroxybenzyl)propanehydrazide. Furthermore, the content of these two impurities increases significantly over time.

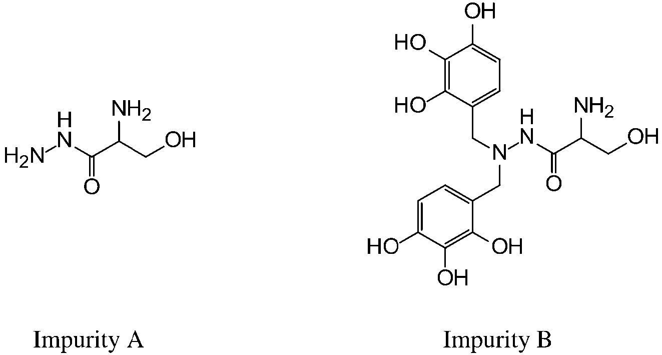

Ma Guizhi et al. (Study on the Influencing Factors of Chemical Stability of Benserazide Hydrochloride [J], China Medical Herald, 2014, 11(35)) discloses the changes in the content of benserazide hydrochloride with temperature and humidity. The results are shown in Table 1 and Table 2.

**Table 1 Effect of Temperature on the Content of Benserazide Hydrochloride**

| Temperature (°C) | 5d | 10d |
|---|---|---|
| 25 | 100.02±0.97 | 100.00±1.31 |
| 40 | 97.89 ±1.54 | 95.43 ±4.57 |
| 60 | 94.33±5.56 | 90.86±5.04 |

**Table 2 Effect of Humidity on the Content of Benserazide Hydrochloride**

| Relative Humidity(%) | 5d | 10d |
|---|---|---|
| 0 | 100.02±1.42 | 100.00±1.19 |
| 60 | 67.43±2.11 | 1.84±0.98 |
| 75 | 32.11±1.13 | 0.00±0.00 |

CN101797144A discloses a co-beneldopa orally disintegrating tablet and preparation therefor. However, currently only conventional preparation of co-beneldopa tablets are available on the domestic market. The orally disintegrating tablets are not marketed and are not clinically available.

CN101623278B discloses a pharmaceutical composition containing levodopa and benserazide hydrochloride. A carrier substance containing benserazide hydrochloride is obtained by spray drying, and then mixed uniformly with other excipients before being compressed into tablets. However, as it uses ethanol solution that is easily evaporated and explosive to prepare the drug-containing carrier via spray drying, which imposes high requirements on production equipment, production facilities, and production operations, it is not conducive to large-scale production.

During the preparation of the co-beneldopa composition, the inventors found that the particle size of calcium hydrogen phosphate significantly affects the stability of the co-beneldopa composition and of the co-beneldopa tablets or capsules prepared therefrom. When the particle size of calcium hydrogen phosphate is too large, the degradation of benserazide hydrochloride accelerates, and the contents of Impurity A and Impurity B increase significantly, thereby adversely affecting the clinical efficacy and safety of the co-beneldopa preparation.

Furthermore, none of the various prior art solutions discloses how to obtain a co-beneldopa composition that is easier to scale up for production and exhibits good stability. Therefore, it is necessary to develop a co-beneldopa composition that is easier to scale up for production and exhibits good stability, and a solid preparation prepared therefrom.

### SUMMARY OF THE INVENTION

To solve the above problem, the present application provides a co-beneldopa composition comprising levodopa, benserazide hydrochloride, and calcium hydrogen phosphate, wherein the calcium hydrogen phosphate has a D50 ≤ 12 µm.

According to an embodiment of the present application, the D50 of the calcium hydrogen phosphate is preferably 8 to 12 µm.

According to an embodiment of the present application, the calcium hydrogen phosphate is anhydrous calcium hydrogen phosphate.

According to an embodiment of the present application, the composition comprises the following components by weight: 200 parts of levodopa, 57 parts of benserazide hydrochloride, and 55 to 275 parts of calcium hydrogen phosphate.

According to an embodiment of the present application, the composition comprises the following components by weight: 200 parts of levodopa, 57 parts of benserazide hydrochloride, and 99 to 102 parts of calcium hydrogen phosphate.

According to an embodiment of the present application, the co-beneldopa composition further comprises other pharmaceutical excipients, which can be selected from one or more of a filler, a binder, a disintegrant, a solubilizer and a lubricant.

The present application further provides a preparation method for co-beneldopa composition, comprising mixing the components contained therein.

According to an embodiment of the present application, the co-beneldopa composition is in solid form, such as powder form or granule form.

The present application also provides the use of the co-beneldopa composition in the manufacture of a medicament for treating Parkinson's disease, Parkinson's syndrome, and restless legs syndrome.

According to an embodiment of the present application, the medicament is a solid preparation, such as a tablet or a capsule.

According to an embodiment of the present application, the tablet may be coated or uncoated. When it is coated, the tablet may comprise a core and a coating layer.

The present application also provides a co-beneldopa tablet comprising the above co-beneldopa composition.

The present application also provides a preparation method for co-beneldopa tablet, comprising compressing the above co-beneldopa composition into a tablet, for example, by direct compression or by granulation followed by tableting, and optionally coating or not coating.

According to an embodiment of the present application, the preparation method for the tablets specifically comprises:
(1) Granulation: mixing levodopa, benserazide hydrochloride, calcium hydrogen phosphate, and one or more of a filler, a binder, a disintegrant, and a solubilizer to obtain granules for tableting;
(2) Tableting: mixing the granules with a lubricant and then tableting;

Optionally, a sieving treatment is performed before and after the granulation.

### Advantageous Effects:

The applicant surprisingly found that by selecting the pharmaceutical composition within the scope of the present application, particularly by selecting anhydrous calcium hydrogen phosphate with a specific particle size as an excipient, the production of benserazide hydrochloride-related Impurity A and Impurity B can be significantly reduced, and the stability of the co-beneldopa composition and related dosage forms can be improved.

Furthermore, when the composition of the present application is applied to solid preparations, especially compressed tablets, the process is simple, stable, and low-cost, making it more suitable for large-scale production. The prepared tablets exhibit good stability without any additional stabilizers or special processes such as coating.

### BRIEF DESCRIPTION OF THE DRAWINGS

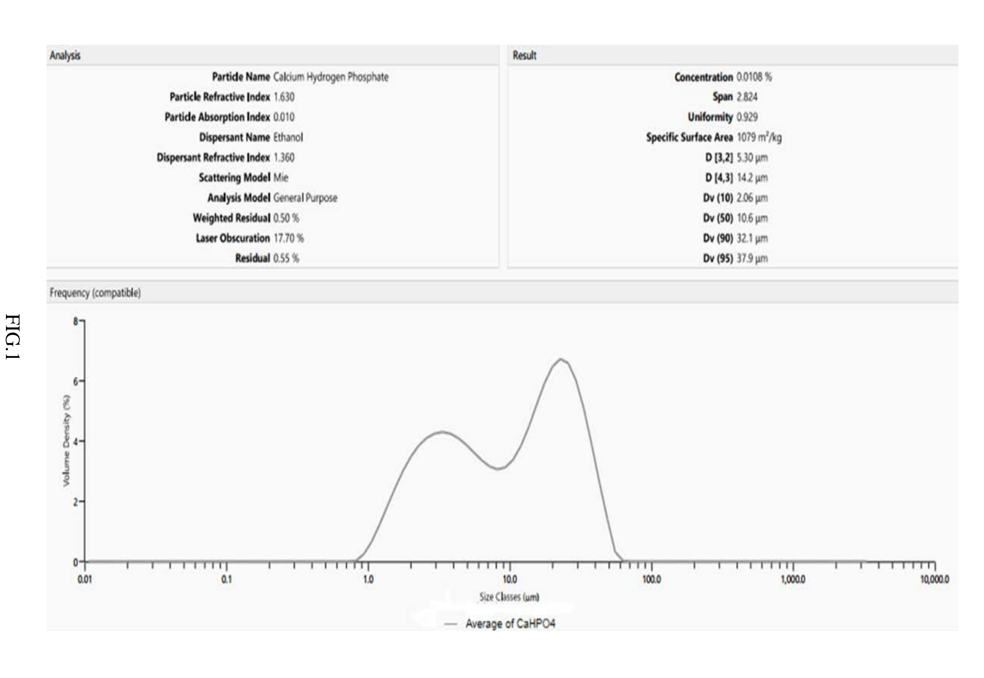

FIG. 1 shows the D50 detection spectrum of the calcium hydrogen phosphate used in Examples 1 and 2.

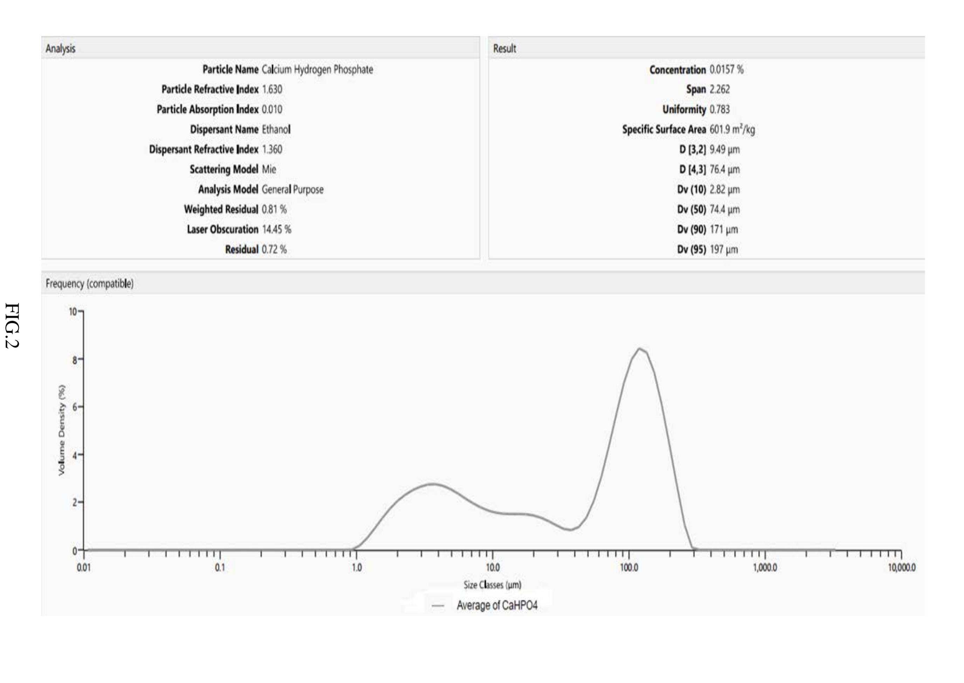

FIG. 2 shows the D50 detection spectrum of the calcium hydrogen phosphate used in Comparative Example 1.

### DETAILED DESCRIPTION

The following detailed description provides further details of the present application.

As used herein, unless otherwise stated, the scientific and technical terms used have the meanings commonly understood by those skilled in the art. Moreover, the laboratory operating steps used herein are all conventional steps widely used in the respective fields.

### Terms:

The terms "having", "comprising" and "including" should be interpreted as open-ended, indicating the presence of the listed elements but not excluding the presence, occurrence, or addition of any other one or more unlisted elements.

As used herein, unless otherwise stated, the term "about" is intended to define the numerical value it modifies, indicating that such value can vary within a certain range. When no range is indicated (e.g., an error range or the standard deviation of the mean given in a graph or data table), the term "about" should be understood as indicating a larger range that includes the stated value, and the range included by rounding to that figure considering significant number, as well as the range including ±10% of the stated value.

All ranges recited herein include the endpoints of those ranges defined as being between two stated values. Whether indicated or not, all values listed herein include the expected experimental error, technical error and instrumental error of the given technology used to measure the value.

In an embodiment of the present application, the amount of levodopa/benserazide hydrochloride in the co-beneldopa composition will generally depend on a therapeutically effective amount.

As used herein, a "therapeutically effective amount" is an amount or quantity of a drug sufficient to elicit the desired or intended therapeutic response, in other words, it is an amount sufficient to cause a significant biological response when administered to a patient.

In an embodiment of the present application, the amount of levodopa/benserazide hydrochloride in the co-beneldopa composition can be: 200 mg/57 mg, 100 mg/28.5 mg.

The anhydrous calcium hydrogen phosphate with D50 ≤ 12 µm in the present application can be directly obtained using preparation methods disclosed in the prior art, or can be obtained by conventional treatment such as micronization of commercially available anhydrous calcium hydrogen phosphate. Preferably, the obtained anhydrous calcium hydrogen phosphate has a D50 of 8 to 12 µm.

According to at least one embodiment of the present application, the D50 of the anhydrous calcium hydrogen phosphate is about 10.6 µm.

According to an embodiment of the present application, the term "filler" means a filler other than anhydrous calcium hydrogen phosphate, and can be selected from one or more of microcrystalline cellulose, mannitol, lactose and corn starch.

In some embodiments, the filler other than anhydrous calcium hydrogen phosphate is microcrystalline cellulose.

In some embodiments, the filler other than anhydrous calcium hydrogen phosphate is mannitol.

In some embodiments, the fillers other than anhydrous calcium hydrogen phosphate are microcrystalline cellulose and mannitol.

According to an embodiment of the present application, the binder can be selected from one or more of pregelatinized starch, ethyl cellulose, methyl cellulose, hydroxypropyl methyl cellulose, carboxymethyl cellulose and polyethylene glycol.

In some embodiments, the binder is pregelatinized starch.

In some embodiments, the binders are pregelatinized starch and ethyl cellulose.

According to an embodiment of the present application, the disintegrant can be selected from one or more of crospovidone, croscarmellose sodium, sodium carboxymethyl starch and sodium alginate.

In some embodiments, the disintegrant is crospovidone.

According to an embodiment of the present application, the solubilizer is selected from one or more of docusate sodium and sodium dodecyl sulfate.

In some embodiments, the solubilizer is docusate sodium.

In some embodiments, the solubilizer is sodium dodecyl sulfate.

According to an embodiment of the present application, the lubricant can be selected from one or more of magnesium stearate, sodium stearyl fumarate, sodium stearate and colloidal silicon dioxide.

In some embodiments, the lubricant is magnesium stearate.

In some embodiments, the lubricants are magnesium stearate and colloidal silicon dioxide.

In some embodiments, the co-beneldopa composition comprises levodopa, benserazide hydrochloride, calcium hydrogen phosphate, microcrystalline cellulose, mannitol, pregelatinized starch, ethyl cellulose, crospovidone, colloidal silicon dioxide, magnesium stearate, and docusate sodium.

In some embodiments, the co-beneldopa composition comprises levodopa, benserazide hydrochloride, calcium hydrogen phosphate, microcrystalline cellulose, mannitol, pregelatinized starch, ethyl cellulose, crospovidone, magnesium stearate, and docusate sodium.

In some embodiments, the co-beneldopa composition comprises levodopa, benserazide hydrochloride, calcium hydrogen phosphate, microcrystalline cellulose, mannitol, pregelatinized starch, crospovidone, colloidal silicon dioxide, magnesium stearate, and docusate sodium.

In some embodiments, the co-beneldopa composition comprises levodopa, benserazide hydrochloride, calcium hydrogen phosphate, microcrystalline cellulose, mannitol, pregelatinized starch, crospovidone, magnesium stearate, and docusate sodium.

In some embodiments, the co-beneldopa composition comprises levodopa, benserazide hydrochloride, calcium hydrogen phosphate, microcrystalline cellulose, mannitol, pregelatinized starch, ethyl cellulose, crospovidone, colloidal silicon dioxide, magnesium stearate, and sodium dodecyl sulfate.

In some embodiments, the co-beneldopa composition comprises levodopa, benserazide hydrochloride, calcium hydrogen phosphate, microcrystalline cellulose, mannitol, pregelatinized starch, ethyl cellulose, crospovidone, magnesium stearate, and sodium dodecyl sulfate.

In some embodiments, the co-beneldopa composition comprises levodopa, benserazide hydrochloride, calcium hydrogen phosphate, microcrystalline cellulose, mannitol, pregelatinized starch, crospovidone, colloidal silicon dioxide, magnesium stearate, and sodium dodecyl sulfate.

In some embodiments, the co-beneldopa composition comprises levodopa, benserazide hydrochloride, calcium hydrogen phosphate, microcrystalline cellulose, mannitol, pregelatinized starch, crospovidone, magnesium stearate, and sodium dodecyl sulfate.

According to an embodiment of the present application, the co-beneldopa composition comprises the following components by weight:

| | |
|---|---|
| Levodopa | 200 parts |
| Benserazide Hydrochloride | 57 parts |
| Calcium Hydrogen Phosphate | 55 to 275 parts |
| Microcrystalline Cellulose | 20 to 60 parts |
| Mannitol | 50 to 150 parts |
| Pregelatinized Starch | 10 to 30 parts |
| Ethyl Cellulose | 0 to 6 parts |
| Crospovidone | 5 to 25 parts |
| Colloidal Silicon Dioxide | 0 to 3 parts |
| Magnesium Stearate | 2 to 8 parts |
| Docusate Sodium or Sodium Dodecyl Sulfate | 0 to 2 parts |

According to a preferred embodiment of the present application, the co-beneldopa composition comprises the following components by weight:

| | |
|---|---|
| Levodopa | 200 parts |
| Benserazide Hydrochloride | 57 parts |
| Calcium Hydrogen Phosphate | 99 to 102 parts |
| Microcrystalline Cellulose | 38 to 42 parts |
| Mannitol | 102 to 104 parts |
| Pregelatinized Starch | 20 parts |
| Ethyl Cellulose | 0 to 3 parts |
| Crospovidone | 10 to 20 parts |
| Colloidal Silicon Dioxide | 0 to 1 part |
| Magnesium Stearate | 5 to 6 parts |
| Docusate Sodium or Sodium Dodecyl Sulfate | 0.2 to 0.3 part |

According to a preferred embodiment of the present application, the co-beneldopa composition comprises the following components by weight:

| | |
|---|---|
| Levodopa | 200 parts |
| Benserazide Hydrochloride | 57 parts |
| Calcium Hydrogen Phosphate | 99 to 102 parts |
| Microcrystalline Cellulose | 38 to 42 parts |
| Mannitol | 102 to 104 parts |
| Pregelatinized Starch | 20 parts |
| Crospovidone | 10 to 20 parts |
| Magnesium Stearate | 5 to 6 parts |
| Docusate Sodium or Sodium Dodecyl Sulfate | 0.2 to 0.3 part |

According to a preferred embodiment of the present application, the co-beneldopa composition comprises the following components by weight:

| | |
|---|---|
| Levodopa | 200 parts |
| Benserazide Hydrochloride | 57 parts |
| Calcium Hydrogen Phosphate | 99 to 102 parts |
| Microcrystalline Cellulose | 38 to 42 parts |
| Mannitol | 102 to 104 parts |
| Pregelatinized Starch | 20 parts |
| Crospovidone | 10 to 20 parts |
| Colloidal Silicon Dioxide | 1 part |
| Magnesium Stearate | 5 to 6 parts |
| Docusate Sodium or Sodium Dodecyl Sulfate | 0.2 to 0.3 part |

According to a preferred embodiment of the present application, the co-beneldopa composition comprises the following components by weight:

| | |
|---|---|
| Levodopa | 200 parts |
| Benserazide Hydrochloride | 57 parts |
| Calcium Hydrogen Phosphate | 99 to 102 parts |
| Microcrystalline Cellulose | 38 to 42 parts |
| Mannitol | 102 to 104 parts |
| Pregelatinized Starch | 20 parts |
| Ethyl Cellulose | 3 parts |
| Crospovidone | 10 to 20 parts |
| Magnesium Stearate | 5 to 6 parts |
| Docusate Sodium or Sodium Dodecyl Sulfate | 0.2 to 0.3 part |

According to a preferred embodiment of the present application, the co-beneldopa composition comprises the following components by weight:

| | |
|---|---|
| Levodopa | 200 parts |
| Benserazide Hydrochloride | 57 parts |
| Calcium Hydrogen Phosphate | 99 to 102 parts |
| Microcrystalline Cellulose | 38 to 42 parts |
| Mannitol | 102 to 104 parts |
| Pregelatinized Starch | 20 parts |
| Ethyl Cellulose | 3 parts |
| Crospovidone | 10 to 20 parts |
| Colloidal Silicon Dioxide | 1 part |
| Magnesium Stearate | 5 to 6 parts |
| Docusate Sodium or Sodium Dodecyl Sulfate | 0.2 to 0.3 part |

According to an embodiment of the present application, the composition further comprises a coloring agent, the coloring agent is selected from one or more of caramel, ferric oxide, grape skin extract, beet red powder, β-carotene, annatto, carmine, turmeric and paprika. In some embodiments, the coloring agent is ferric oxide, present in an amount of 1 to 2 parts by weight in the composition.

The co-beneldopa composition of the present application can be formulated into various dosage forms using any shape or form known in the filed of pharmaceutical science. The dosage form of the present application can be a tablet or a capsule. The dosage form may further comprise surface markings, cuts, grooves, letters, and/or numbers for decoration, identification, and/or other purposes.

According to an embodiment of the present application, in the preparation method of the tablet, a sieving treatment may optionally be performed before mixing one or more of levodopa, benserazide hydrochloride, calcium hydrogen phosphate,a filler, a binder, a disintegrant and a solubilizer, and also after granulation.

According to an embodiment of the present application, the granulation in the preparation method of the tablet may be dry granulation or wet granulation.

As is commonly done in the art, the dosage form may comprise a finishing coat to provide desired gloss, color, taste, or other aesthetic properties.

The following examples are intended to illustrate the present application without limiting its scope in any way.

The benserazide hydrochloride used in the specific examples of the present application was the same batch of self-prepared active pharmaceutical ingredient. The anhydrous calcium hydrogen phosphate with a D50 of about 74.4 µm was a commercially available product. After treatment, anhydrous calcium hydrogen phosphate with a D50 of about 10.6 µm was obtained.

### Comparative Example 1:

Prescription composition: The content of each component per co-beneldopa tablet is shown in Table 3, wherein the anhydrous calcium hydrogen phosphate has a D50 of about 74.4 µm.

**Table 3**

| Component | Formula Amount (mg/tablet) |
|---|---|
| Levodopa | 200.0 |
| Benserazide Hydrochloride | 57.0 |
| Anhydrous Calcium Hydrogen Phosphate | 100.0 |
| Mannitol | 103.2 |
| Microcrystalline Cellulose | 41.6 |
| Pregelatinized Starch | 20.0 |
| Crospovidone | 20.0 |
| Colloidal Silicon Dioxide | 1.0 |
| Sodium Lauryl Sulfate | 0.2 |
| Ferric Oxide | 1.5 |
| Magnesium Stearate | 5.5 |

Preparation process: Levodopa and benserazide hydrochloride were uniformly mixed with other excipients except magnesium stearate and subjected to a dry granulation. The resulting dry granules were then uniformly mixed with magnesium stearate and added into a tablet machine and compressed into tablets.

### Example 1:

Prescription composition: The content of each component per co-beneldopa tablet is shown in Table 4, wherein the anhydrous calcium hydrogen phosphate has a D50 of about 10.6 µm.

**Table 4**

| Component | Formula Amount (mg/tablet) |
|---|---|
| Levodopa | 200.0 |
| Benserazide Hydrochloride | 57.0 |
| Anhydrous Calcium Hydrogen Phosphate | 100.0 |
| Mannitol | 103.2 |
| Microcrystalline Cellulose | 41.6 |
| Pregelatinized Starch | 20.0 |
| Crospovidone | 20.0 |
| Colloidal Silicon Dioxide | 1.0 |
| Sodium Dedocyl Sulfate | 0.2 |
| Ferric Oxide | 1.5 |
| Magnesium Stearate | 5.5 |

Preparation process: Levodopa and benserazide hydrochloride were uniformly mixed with other excipients except magnesium stearate and subjected to a dry granulation. The resulting dry granules were then uniformly mixed with magnesium stearate and added intio a tablet machine and compressed into tablets.

### Example 2:

Prescription composition: The content of each component per co-beneldopa tablet is shown in Table 5, wherein the anhydrous calcium hydrogen phosphate has a D50 of about 10.6 µm.

**Table 5**

| Component | Formula Amount (mg/tablet) |
|---|---|
| Levodopa | 200.0 |
| Benserazide Hydrochloride | 57.0 |
| Anhydrous Calcium Hydrogen Phosphate | 101.5 |
| Mannitol | 103.2 |
| Microcrystalline Cellulose | 38.6 |
| Pregelatinized Starch | 20.0 |
| Crospovidone | 20.0 |
| Ethyl Cellulose | 3.0 |
| Docusate Sodium | 0.2 |
| Colloidal Silicon Dioxide | 1.0 |
| Magnesium Stearate | 5.5 |

Preparation process: Levodopa, benserazide hydrochloride, and other excipients except magnesium stearate were subjected to a wet granulation process and dried to obtain dry granules. The resulting dry granules were then uniformly mixed with magnesium stearate and added into a tablet machine and compressed into tablets.

### Stability Test:

The tablets obtained from Comparative Example 1 and Examples 1-2 were packaged in conventional blister packs and placed under conditions of 60 C and RH 75% for 5 days to conduct a stability test. The changes in the content of benserazide hydrochloride under high temperature and high humidity conditions were investigated. The content determination method described in the Levodopa and Benserazide Tablets monograph of Part II of the Chinese Pharmacopoeia 2020 edition was used for detection and anylysis. The results are shown in Table 6.

**Table 6 Changes in the Content of Benserazide Hydrochloride, Impurity A, and Impurity B in Co-Beneldopa Tablets after 5 Days**

| **Sample** | **Benserazide Hydrochloride** | **Impurity A** | **Impurity B** |
|---|---|---|---|
| Comparative Example 1 | 85.6% | 6.00% | 6.60% |
| Example 1 | 100.0% | 0.18% | 0.12% |
| Example 2 | 98.7% | 0.39% | 0.97% |

The results indicate that the co-beneldopa tablets prepared using anhydrous calcium hydrogen phosphate with a D50 of about 10.6 µm exhibited significantly improved stability. The degradation of benserazide hydrochloride was inhibited, and the contents of Impurity A and Impurity B were significantly reduced.

### Other Embodiments

The present disclosure also provides embodiments in which any of the above embodiments may be combined with any one or more of these embodiments, provided that the combination is not mutually exclusive. The present disclosure further provides uses for treating the indications disclosed herein or one or more symptoms thereof, and uses in the manufacture of a medicament for treating the indications disclosed herein or one or more symptoms thereof, the scope of which is equivalent to any of the embodiments disclosed above, or any combination of embodiments that are not mutually exclusive. Although the present disclosure has been described with reference to specific details of certain embodiments in the above examples, it is understood that modifications and variations are encompassed within the spirit and scope of the present disclosure.

## Claims

1. A co-beneldopa composition comprising levodopa, benserazide hydrochloride, and calcium hydrogen phosphate, **characterized in that** the calcium hydrogen phosphate has a D50 ≤ 12 µm, preferably, the calcium hydrogen phosphate has a D50 of 8 to 12 µm.

2. The composition according to claim 1, **characterized in that** the calcium hydrogen phosphate is anhydrous calcium hydrogen phosphate.

3. The composition according to claim 1 or 2, **characterized in that** the composition comprises the following components by weight: 200 parts of levodopa, 57 parts of benserazide hydrochloride, and 55 to 275 parts of calcium hydrogen phosphate; preferably, 99 to 102 parts of calcium hydrogen phosphate.

4. The composition according to any one of claims 1-3, **characterized in that** the composition further comprises one or more of a filler, a binder, a disintegrant, a solubilizer and a lubricant.

5. The composition according to any one of claims 1-4, **characterized in that**:
the filler is selected from one or more of microcrystalline cellulose, mannitol, lactose and corn starch, preferably selected from one or more of microcrystalline cellulose and mannitol;
the binder can be selected from one or more of pregelatinized starch, ethyl cellulose, methyl cellulose, hydroxypropyl methyl cellulose, carboxymethyl cellulose and polyethylene glycol, preferably selected from one or more of pregelatinized starch and ethyl cellulose;
the disintegrant can be selected from one or more of crospovidone, croscarmellose sodium, sodium carboxymethyl starch and sodium alginate, preferably crospovidone;
the solubilizer is selected from one or more of docusate sodium and sodium dodecyl sulfate;
the lubricant is selected from one or more of magnesium stearate, sodium stearyl fumarate, sodium stearate and colloidal silicon dioxide, preferably selected from one or more of magnesium stearate and colloidal silicon dioxide.

6. The composition according to any one of claims 1-5, **characterized in that** the composition comprising the following components by weight:
| | |
|---|---|
| Levodopa | 200 parts |
| Benserazide Hydrochloride | 57 parts |
| Calcium Hydrogen Phosphate | 55 to 275 parts |
| Microcrystalline Cellulose | 20 to 60 parts |
| Mannitol | 50 to 150 parts |
| Pregelatinized Starch | 10 to 30 parts |
| Ethyl Cellulose | 0 to 6 parts |
| Crospovidone | 5 to 25 parts |
| Colloidal Silicon Dioxide | 0 to 3 parts |
| Magnesium Stearate | 2 to 8 parts |
| Docusate Sodium or Sodium Lauryl Sulfate | 0 to 2 parts |

7. Use of the composition according to any one of claims 1-6 in the manufacture of a medicament for treating Parkinson's disease, Parkinson's syndrome, and restless legs syndrome.

8. The use according to claim 7, **characterized in that** the medicament is a solid preparation, preferably a tablet or a capsule.

9. A preparation method for a co-beneldopa tablet, **characterized by** comprising compressing the composition according to any one of claims 1-6 into a tablet.

10. The preparation method according to claim 9, **characterized in that** the preparation method specifically comprises:
(1) granulation: mixing levodopa, benserazide hydrochloride, calcium hydrogen phosphate, and one or more of a filler, a binder, a disintegrant, and a solubilizer to obtain granules for tableting;
(2) tableting: mixing the granules with a lubricant and then tableting;
optionally, a sieving treatment is performed before and after the granulation.
